## Europäisches Patentamt

## European Patent Office

## Office européen des brevets

(11) Veröffentlichungsnummer: **0 120 340**
**B1**

---

(12) **EUROPÄISCHE PATENTSCHRIFT**

(45) Veröffentlichungstag der Patentschrift :
**12.11.86**

(21) Anmeldenummer : **84102201.5**

(22) Anmeldetag : **01.03.84**

(51) Int. Cl.⁴ : **C 23 F 1/00, G 01 N 1/32**

---

(54) Verfahren zum Erkennen von Gefügeinhomogenitäten in Titanlegierungsproben und Schweisslingen.

---

(30) Priorität : **16.03.83 DE 3309448**

(43) Veröffentlichungstag der Anmeldung :
**03.10.84 Patentblatt 84/40**

(45) Bekanntmachung des Hinweises auf die Patenterteilung : **12.11.86 Patentblatt 86/46**

(84) Benannte Vertragsstaaten :
**AT BE CH DE FR GB IT LI NL SE**

(56) Entgegenhaltungen :
**EP-A- 0 035 241**
**EP-A- 0 072 986**
**FR-A- 2 142 079**
**US-A- 3 108 919**
**US-A- 3 891 456**
**METAL FINISHING ABSTRACTS, Band 23, Nr. 1, Januar/Februar 1981, Seite 7B, Teddington, Middlesex, GB;**
**Schumann: Metallographie, (1974) VEB Deut. Verlag für Grundstoffindustrie S. 17, 19, 275 593**

(73) Patentinhaber : **MTU MOTOREN- UND TURBINEN-UNION MÜNCHEN GMBH**
**Dachauer Strasse 665 Postfach 50 06 40**
**D-8000 München 50 (DE)**

(72) Erfinder : **Thoma, Martin, Dr.-rer-nat.**
**Giselastrasse 3**
**D-8000 München 40 (DE)**

**Beschreibung**

Die Erfindung betrifft ein Kontrastierverfahren zum Erkennen von Seigerungen in hochbeanspruchten Bauteilen aus. Titanlegierungen, welche, wenn sie Inhomogenitäten in der Form von Seigerungen enthalten, zum Bauteilversagen führen. Hochbeanspruchte Bauteile wie z. B. Verdichterscheiben, Verdichterwellen und Abstandsringe in Turbomaschinen müssen aber frei von Seigerungen sein. Die Seigerungen können Bereiche hoher Härte sein, hervorgerufen durch Sauerstoffanreicherung. Es gibt aber auch weiche β-Seigerungen, die z. B. große Bereiche von β-Legierungsgehalt darstellen.

In der Metallographie werden Schliffherstellungen zur Gefügeuntersuchung bereits seit langer Zeit angewandt. Bekannt sind dabei vor allem zwei Verfahren : elektrochemisch durch Analysieren in einem Elektrolyten oder durch Ätzungen mit verschiedenen Chemikalien wie Salzsäure, Schwefelsäure, Flußsäure oder ähnlichem, sogenannte Tiefätzungen. In die dabei entstehenden Riefen läßt man einen Indikator wie Farbindikator eindringen. Ein Eindring-Indikator wird bei entsprechender Beleuchtung insbesondere mit polarisierendem oder fluoreszierendem Licht die tiefen Risse oder Riefen sichtbar werden lassen. Ein solches Verfahren ist zum Beispiel in der offengelegten französischen Patentanmeldung 2 142 079 beschrieben. Chemische Lösungen zur Tiefenätzung von Titanoberflächen sind auch in der veröffentlichten europäischen Patentanmeldung 0 035 241 und 0 072 986 beschrieben. Beide Schriften betreffen Tiefenätzverfahren zur Aktivierung von Titanoberflächen, wobei die Werkstoffoberfläche in ihrer Rauhigkeit vergrößert werden soll, damit sie für das nachfolgende Plattieren mit metallischen Überzügen eine verbesserte Haftung ergibt.

Beiden europäischen Veröffentlichungen ist gemeinsam, daß bei diesen Verfahren zwar eine Wasserstoffaufnahme des Grundwerkstoffs, die ebenfalls zur Versprödung führt, ausgeschlossen werden soll, unberücksichtigt blieb jedoch die Möglichkeit von Seigerungen, hervorgerufen durch Sauerstoff- oder Stickstoffanreicherung. Die Veröffentlichungen bieten daher für die Aufgabe der Erfindung keine Lösung. Für hochbeanspruchte Bauteile aus Titanlegierungen wie Verdichterscheiben, Wellen, Ringe oder dergleichen — einschließlich geschweißter Bauteile — lassen sich die bekannten Verfahren zur Schliffherstellung und zum chemischen Abtragen nicht anwenden aus mehreren Gründen :

Das fertige Bauteil oder Werkstück muß sein Endmaß behalten und darf nicht mehr abgeschliffen oder chemisch abgetragen werden.

Gefertigte Bauteile sind überwiegend nicht planparallel und daher ist eine gleichmäßige Abtragung äußerst schwierig.

Beim Entfetten, was insbesondere im Bereich einer Schweißstelle schon nötig sein kann, wenn durch Angreifen der Werkstücke Handschweiß zurückblieb, ist es nicht gleichgültig, welche Entfettung gewählt wird. Bei Titanlegierungen hat man bisher wegen der Gefahr von Spannungsrißkorrosion von einer Verwendung von Trichlorethen abgeraten.

Beim mechanischen Abtragen wie Schmiergeln, Schleifen oder ähnlichen Vorbehandlungen ist zu beachten, daß Titan oder Titanlegierungen leicht oberflächlich verformt und verschmiert werden. Deshalb muß ein äußerst feinkörniges und schonendes Behandlungsverfahren und -material angewendet werden.

Aufgabe der Erfindung ist daher die Schaffung eines Verfahrens, bei dem große Kontraste erzielbar sind, so daß Seigerungen des Gefüges in hochbeanspruchten Bauteilen aus Titanlegierungen auf einfache Weise auch ohne Indikator oder durch besonderes Licht sichtbar gemacht werden können. Gleichermaßen soll die Erfindung zur Beurteilung von Schweißstellen an solchen Bauteilen geeignet sein. Endmaße müssen eingehalten werden.

Gelöst wird die, der Erfindung zugrunde liegende Aufgabe durch ein Verfahren der in Anspruch 1 genannten Art. Dabei wurden gute Ergebnisse im Sinne der Aufgabenstellung vor allem dann erzielt, wenn der Dampfentfettungsvorgang in Trichlorethen erfolgte, das Aluminiumoxid mit einem Druck von 4 bis 5 bar gestrahlt wurde und das abtragende Ätzen mit einer Salpetersäure-Flußsäurelösung durchgeführt wurde, deren Konzentration 400 g/l $HNO_3$ und 5 g/l HF aufwies, wobei die Ätzdauer zwischen 2 und 20 Minuten lag. Das Gefügeätzen findet vorzugsweise bei einer Temperatur von 40 bis 80 °C in Bädern statt, wie sie in den Patentansprüchen 4 und 5 angegeben sind.

Besonders vorteilhaft ist es, wenn beim Ätzvorgang gemäß Merkmal b) des Hauptanspruchs die in Anspruch 2 angegebene Lösung verwendet wird.

Erfindungsgemäß ist demnach ein Verfahren zum Erkennen von Seigerungen in hochbeanspruchten Bauteilen aus Titanlegierungen angegeben, das eine besonders gute Kontrastierung zur Folge hat. Die erzeugte Oberfläche wird mattgrau und die Seigerungen heben sich silbrig glänzend von der grauen Oberfläche ab. Gleichzeitig wird die α/β-Gefügeverteilung sichtbar gemacht.

Besonders vorteilhaft ist die Anwendung der Erfindung bei Schweißstellen in hochbeanspruchten Bauteilen aus Titanlegierungen. Nach dem erfindungsgemäßen Behandeln des Schweißlings läßt sich die Schweißstelle daran leicht beurteilen.

Im folgenden werden Beispiele eines erfindungsgemäßen Seigerungsätzverfahrens widergegeben :

Die zu untersuchende Probe wird vorab in Trichlorethen einem Dampfentfettungsvorgang unterzogen. Anschließend wird die Probe mit $Al_2O_3$/270 mesh lichte Maschenweite 0,053 mm bei einem Druck von 4 bis 5 bar gestrahlt. Der sich anschließende Beizvorgang dauert 15 Minuten und erfolgt in einer $HNO_3/HF$-Lösung bei einer Konzentration von 400 g pro 5 g. Nach einem sich anschließenden Wasserspülungsvorgang erfolgt das Gefügeätzen, und zwar in einer $CrO_3/H_2SiF_6/HF$-Lösung bei einem Molverhältnis 1,8 : 0,6 : 0,3 mol.

Die Probe wird anschließend gespült und getrocknet. Es hat sich gezeigt, daß nach 15-minütigem Beizen in der $HNO_3/HF$-Lösung ein Abtrag von 2-4 μm vorhanden war. Der Abtrag nach dem Ätzvorgang in der Ätzlösung $CrO_3/HF/H_2SiF_6$ betrug ca. 2 μm.

## Patentansprüche

1. Kontrastierverfahren zum Erkennen von Seigerungen in hochbeanspruchten Bauteilen aus Titanlegierungen, wobei die Bauteile
   a) gereinigt werden durch einen Dampfentfettungsvorgang mit Trichlorethen,
   b) die Oberfläche unter Druck mit $Al_2O_3/270$ mesh (lichte Maschenweite 0,053 mm) gestrahlt wird,
   c) das abtragende Ätzen mit einer Salpetersäure-/Flußsäurelösung durchgeführt wird und
   d) Spülen mit Wasser folgt,
   e) Gefügeätzen mit einer Lösung aus Chromsäure, Flußsäure und Arsen- oder Antimon- oder Siliziumverbindungen, wobei folgende Molverhältnisse vorliegen :
   $8 \geq F/Sb \geq 5$ bzw.
   $8 \geq F/Si \geq 5$ bzw.
   $8 \geq F/As \geq 5$
   $6 \geq Cr/(Sb) \geq 3$
   bzw. $Cr/(As)$
   bzw. $Cr/(Si)$
   und ferner ein Molkonzentrationsbereich von 0,1-2 Mol/l Si bzw. Sb bzw. As eingehalten wird,
   f) Spülen mit Wasser.

2. Verfahren nach Anspruch 1, dadurch gekennzeichnet, daß die Salpetersäure-Flußsäurelösung für das abtragende Ätzen eine Konzentration von 400 g/l $HNO_3$ und 5 g/l HF aufweist.

3. Verfahren nach Anspruch 1 oder 2, dadurch gekennzeichnet, daß das Gefügeätzen bei einer Temperatur von 40 bis 80 °C stattfindet.

4. Verfahren nach den Ansprüchen 1 bis 3, dadurch gekennzeichnet, daß bei einem Ätzvorgang eine $CrO_3/HF/H_2SiF_6$-Lösung verwendet wird, wobei die prozentualen Anteile von $CrO_3$, HF und $H_2SiF_6$ entsprechend der im Anspruch 1 angegebenen Molverhältnisse und der Molkonzentration gewählt werden.

5. Verfahren nach Anspruch 4, dadurch gekennzeichnet, daß die Komponente $H_2SiF_6$ ersetzt wird durch $Sb_2O_3$ oder $SbF_3$ oder $AsO_3$, wobei die prozentualen Anteile von $CrO_3$, HF und $Sb_2O_3$, $SbF_3$ oder $AsO_3$ entsprechend den im Anspruch 1 angegebenen Molverhältnissen und der Molkonzentration gewählt werden.

6. Anwendung des Verfahrens nach den Ansprüchen 1 bis 5 zum Erkennen von Seigerungen in hochbeanspruchten Bauteilen aus Titanlegierungen, um daran Schweißstellen insgesamt beurteilen zu können.

## Claims .

1. Contrasting method for detecting liquations in highly stressed components made of titanium alloys, whereby the components are subjected to
   a) cleaning by a steam degreasing method using trichloroethane ;
   b) blasting of the surface thereof under pressure with $Al_2O_3/270$ mesh (clear mesh width 0.053 mm) ;
   c) stripping etching with a solution containing nitric and hydrofluoric acids ; and
   d) rinsing with water ;
   e) texture etching with a solution containing chromic acid, hydrofluoric acid, and arsenic or antimony or silicon compounds, wherein the following molar relationships apply
   $8 \geq F/Sb \geq 5$ or
   $8 \geq F/Si \geq 5$ or
   $8 \geq F/As \geq 5$
   $6 \geq Cr/(Sb) \geq 3$ or
   $Cr/(As)$ or
   $Cr/(Si)$
   and further within the molar concentration range 0.1 to 2 mol/l Si or Sb or As ;
   f) rinsing with water.

2. A method according to claim 1, characterised in that the nitric acid-hydrofluoric acid solution for carrying out the stripping etching has a concentration of 400 g/l $HNO_3$ and 5 g/l HF.

3. A method according to claim 1 or claim 2, characterised in that the texture etching is carried out at a temperature of from 40 to 80 °C.

4. A method according to any of claims 1 to 3, characterised in that a solution of $CrO_3/HF/H_2SiF_6$ is used for the etching process, in which the percentage parts of the components $CrO_3$, HF, and $H_2SiF_6$ are selected so as to correspond to the molar relationships and molar concentration defined in claim 1.

5. A method according to claim 4, characterised in that the component $HSiF_6$ is replaced by $Sb_2O_3$ or $AsO_3$, wherein the percentage of the components of $CrO_3$, HF and $Sb_2O_3$, $SbF_3$ or $AsO_3$ are selected so as to correspond to the molar relationships and molar concentration defined in claim 1.

6. Use of the method according to claims 1 to 5 for the detection of liquations in highly-stressed components made of titanium alloys, in order to evaluate welding seams as a whole.

## Revendications

1. Procédé de contrastage pour détecter des ségrégations dans des pièces très sollicitées réalisées dans des alliages au titane, procédé selon lequel :
   a) on nettoie les pièces dans du trichloréthylène au cours d'un procédé de dégraissage à la vapeur,

b) on pulvérise sur la surface et sous pression, de la poudre $Al_2O_3$ d'une granulométrie de 270 mailles (ouverture des mailles égales à 0,053 mm);

c) on effectue l'attaque chimique d'enlèvement dans une solution à acide nitrique/acide fluorhydrique et,

d) on rince avec de l'eau,

e) l'attaque du réseau se fait avec une solution d'acide chromique, d'acide fluorhydrique et de combinaisons d'arsenic ou d'antimoine ou aussi de silicium selon les rapports molaires suivants :

$8 \geqq F/Sb \geqq 5$ ou
$8 \geqq F/Si \geqq 5$ ou
$8 \geqq F/As \geqq 5$
$6 \geqq Cr/(Sb) \geqq 3$
ou $Cr/(As)$
ou $Cr/(Si)$

et en outre on respecte une plage de concentration molaire de 0,1-2 mol/l Si ; Sb ; As,

f) on rince avec de l'eau.

2. Procédé selon la revendication 1, caractérisé en ce que la solution acide nitrique/acide fluorhydrique présente une concentration de 400 g/l $HNO_3$ et de 5 g/l HF pour l'attaque chimique d'enlèvement.

3. Procédé selon la revendication 1 ou 2, caractérisé en ce que l'attaque du réseau se fait à une température comprise entre 40 et 80 °C.

4. Procédé selon les revendications 1 à 3, caractérisé en ce que pour une opération d'attaque chimique, on utilise une solution $CrO_3/HF/H_2SiF_6$, la teneur en pourcentage de $CrO_3$, HF et $H_2SiF_6$ correspondant aux rapports molaires indiqués à la revendication 1 et à la concentration molaire.

5. Procédé selon la revendication 4, caractérisé en ce que le composant $H_2SiF_6$ est remplacé par $Sb_2O_3$ ou $SbF_3$ ou $AsO_3$, la teneur en pourcentage de $CrO_3$, HF et $Sb_2O_3$, $SbF_3$ ou $AsO_3$ correspondant aux rapports molaires donnés à la revendication 1 et la concentration molaire.

6. Application du procédé selon les revendications 1 à 5 pour détecter des ségrégations dans des pièces très sollicitées, réalisées en alliages au titane pour pouvoir apprécier globalement les soudures.